# EUROPEAN PATENT APPLICATION

(11) **EP 4 014 905 A1**
(43) Date of publication of application: **22.06.2022**
(21) Application number: 20215218.7
(22) Date of filing: 17.12.2020
(51) Int. Cl.: A61B 17/86, A61B 17/88

(54) **STABILIZATION OF TWO ADJACENT BONES**

(71) Applicant: LfC Spólka z o.o., 65-364 Zielona Góra (PL)
(72) Inventor: Ciupik, Lechoslaw Franciszek, 66-016 Czerwiensk (PL)
(74) Representative: PATERIS Patentanwälte PartmbB

(57) **Abstract**

An implant is provided which enhances structural stability across a joint between a first and a second bone to be immobilized. The implant comprises a first and a second element, wherein the first element comprises a proximal, a distal and an intermediate section. The front end of the implant is formed as a pointed head with structures enabling the implant to penetrate bone material, including a channel-like structure running from the front end to a reservoir in the intermediate section. The reservoir is provided for receiving bone material through the channel-like structure and distributing it into a space between two bones upon placing the implant into the bones. A tool is provided for placing the implant into two bones, wherein the tool has means for connecting it to structures in the first element and the second element, so that the two elements can be moved independently.

## Description

The present invention refers to an implant for immobilizing a joint between opposing bones and, more particularly, to a sacroiliac implant. Furthermore the present invention refers to a tool for placing the implant and to a system comprising the implant and the tool.

The sacroiliac joint is subject to arthritic and aging changes like other arthrodial joints. Disorders of the sacroiliac joint can cause low back pain and radiating buttock and leg pain. Surgical treatment of these disorders may include immobilization of a joint. A number of prior art devices exist which may be used to stabilize or to fuse bones. This number comprises devices which are installed within the sacrum and the ilium.

The document EP1029519 discloses an implant which facilitates enhanced structural stability across a joint to be immobilized. The sacroiliac implant is an elongated structure having two cylindrical sections of differing diameters. The implant further has like-handed, but dissimilar pitched threaded sections at the proximal and distal end thereof to produce compressive forces across the joint. A hollow core traverses the length of the implant. A plurality of apertures or openings is formed through the implant transversely to the longitudinal axis, which communicate with the hollow core. The openings or apertures provide a conductive path of healing for bony ingrowth. Means are provided on the proximal end of the implant for engagement with a tool to drive the implant.

The document US 2009/0099610 discloses methods of stabilizing the sacroiliac joint by placing an expandable device in the joint to generate laterally opposing forces against the iliac and sacral surfaces of the sacroliliac joint in securely seat the device in a plane generally parallel to the sacroiliac joint. The expandable device is coated with or otherwise contains a bone material to promote fusion of the joint. The expandable device can be, for example, an expandable cage, a balloon-expandable stent or a self-expanding stent.

The document US 2011/0034957 discloses a bone anchoring device, which includes a bone anchoring element having a shank and a head, a continuous on-piece first locking element and a second locking element. The receiver member has a first chamber for receiving the dead, the dead being pivotable in the first chamber, and a second chamber with a channel for receiving the rod. A first locking element fixes the head in the first chamber at an angel, and a second locking element fixes the rod in the second chamber. The receiver member includes a body including both the first chamber and the second chamber. The head and the rod can be inserted a locked independently from each other. The head is fixed by exerting pressure onto it. The bone anchoring device is particularly applicable to the stabilization of the spine in the sacro-iliac and the cervical region.

The document US 2011/184519 discloses a sacro-iliac implant delivery tool including a first arm connected to a first cannula. The first cannula is rotatable relative to the first arm and is configured for support of a fastening element. A second arm is connected to the first arm. The second arm is rotatable relative to the first arm and is configured for detachable connection to a sacro-iliac implant. The second arm and the first cannula are configured for rotation relative to the first arm to a preset orientation such that the second arm is disposed along a first predetermined trajectory for delivering the sacro-iliac implant and the first cannula is disposed along a second predetermined trajectory for delivering the fastening element in alignment with the sacro-iliac implant for transarticular fixation.

The document US 2011/0184520 discloses a sacro-iliac implant system including at least one implant including a body defining an outer surface and being expandable in at least one dimension. The body is configured to expand in the at least one dimension from a first orientation to a second orientation such that the outer surface engages and spaces apart opposing articular surfaces of a sacro-iliac joint to fix the body with the articular surfaces.

The document US 2012/191191 discloses a sacro-iliac implant including a body extending from a first portion having an outer surface configured for fixation with a sacrum to a second portion having an outer surface being spaced apart and non-continuous with the outer surface of the first portion. A sleeve is disposed about the body and configured for implantation within at least an ilium. The sleeve extends from a first portion to a second portion having an inner surface and a flange disposed to engage an outer non-articular surface of the ilium. The inner surface of the second portion of the sleeve is engageable with the outer surface of the second portion of the body to cause axial translation of the body relative to the sleeve such that naturally separated articular surfaces of the sacrum and ilium are drawn into fixation to immobilize the sacroiliac joint.

The document US 2013/172736 discloses methods and apparatus for diagnostic and/or therapeutic manipulation of the sacroiliac joint. The sacroiliac joint may be immobilized by forming the fusion mass forms using at least one fixation member. In one embodiment, the fixation member comprises an external member and two internal members which threadedly engage each other. The fixation member is configured to expand and comprises bone growth promoting feature. The expanded member fixates the ilium to the sacrum on the side of the target sacroiliac joint, thus the sacroiliac joint is immobilized and fused.

The document US 2013/245703 discloses a fixation device for sacroiliac joint stabilization. The fixation device includes an elongated body comprising a bone anchor at a distal end. An axially moveable proximal anchor is carried by the proximal end of the fixation device. In one embodiment, the device is inserted through the ilium of the pelvis and the bone anchor is rotated into position within the sacrum. The proximal anchor is distally advanced with respect to the bone anchor to provide compression across the sacroiliac joint. The document WO 2014/018240 discloses a sacro-iliac implant including an inner member having an inner surface and an outer surface. The inner member extends between a first end and a second end configured for penetrating a sacrum. An outer member extends between a first end including a flange and being configured to engage an outer non-articular surface of an ilium and a second end. The inner member is rotatable relative to the outer member such that the outer surface of the inner member adjacent its first end engages the outer member to cause axial translation of the inner member relative to the outer member in a configuration such that separated articular surfaces of the sacrum and the ilium are drawn into fixation.

It is one general objective of the present invention to provide an advantageous implant for stabilization and fusion of two adjacent bones, particularly of a sacrum and an ilium.

The first objective is solved by an implant according to claim 1. Further advantageous embodiments of the invention are contained in the dependent claims, the figures and the examples.

A first aspect of the invention is related to an implant for immobilizing a joint between a first bone and a second bone, comprising at least a first element and a second element, wherein the first element has a cylindrical body comprising:
a proximal section having a first thread,
a distal section having a second thread, and
an intermediate section interconnecting said proximal and distal sections, wherein the proximal, distal and intermediate section have an external surface,
wherein the front end of the distal section is formed as a cutting head comprising at least one cutting blade with at least one cutting edge,
wherein the second element has a cylindrical body with an external surface having a third thread and an internal surface having a fourth thread,
wherein the pitch of the fourth thread is of the same size as the pitch of the first thread, and the diameter of the internal surface of the second element is the same as the diameter of the external surface of the first element.

The inventive implant is advantageous because it facilitates the stabilization and fusion of two adjacent bones, particularly of a sacroiliac joint. It can also be advantageously used for a bone elongation or bone abridgement. A further advantage of the inventive implant is that the implant can be placed by use of one single tool.

The design of the second element is advantageous because the third thread facilitates a setting of the implant by screwing of the second element into the second bone, in addition to the screwing of the first element into the first bone which is facilitated by the second thread. Moreover, the second element can be moved relatively to the first element by means of the fourth thread functioning as an internal thread mating with the complementary first thread.

The cutting blades facilitate advantageously the movement of the implant, particularly of the first element, into the bone material by screwing, thereby removing bone material. The front end of the second element has ideally advantageous cutting blades, as well, for cutting bone and drilling the second element into the bone material.

The recess forming the reservoir is a hollow or a depression in the external surface of the intermediate section of the implant.

In the inventive implant, the second thread and the third thread have the same pitch. This design is advantageous because it facilitates the implantation of the implant by way of screwing, and because the implant can be screwed out and screwed back in again at the same site, without disrupting the formed joint between the adjacent bones. Compressing the adjacent bones or pushing them apart can be done by laterally moving the first element relative to the second element. The lateral movement can be induced through the interaction of the first and fourth threads by rotating the first element relative to the second element.

In another preferred embodiment, the second thread has a pitch which is different from the pitch of the third thread. The thread with the higher pitch allows fewer rotations per axial length of the implant as compared to the thread with the lower pitch. The different pitches advantageously allow a movement of two adjacent bones relatively to each other upon an implantation process. When the implant is turned in one direction for screwing the implant into the bones, the design with the different pitches allows a movement of the two adjacent bones towards each other, which is advantageous upon an implantation process. When the implant is turned into the other direction, the two adjacent bones are moved away from each other, which is advantageous in applications in which bones are to be separated. The two adjacent bones can also be moved by way of the relative movement of the first and second elements, as explained above.

It is preferred if the intermediate section of the first element has at least one recess in the external surface forming a reservoir suitable for bone material which is separated from a bone upon an implantation process. Upon an implantation process, the reservoir can receive bone material removed from the bone by the cutting head at the front of the first element, and bone material removed by the second element, as well, which will be pushed by the second element towards the reservoir.

It is preferred if the reservoir in the intermediate section comprises means which are formed for directing a flow of material from the reservoir into the space between the first and second bone. These means are structures which are directing the movement of the bone material from the reservoir into the space between the bones upon an implantation procedure. The means are advantageous because the distribution of bone material into the space between the first and second bone supports the immobilization of the joint between the first and second bone.

It is further preferred if the first element comprises at least one channel-like structure is running from the front end to the reservoir. The channel-like structure is advantageous because bone material, separated from the bone upon an implantation process, can be transported away from the front end of the first element through the channel-like structure.

The channel-like structure has ideally a helical form which is crossing the ridges of the second thread. This form advantageously supports the transport of bone material from the front end. It can, however, also have a straight form which is running parallel to the longitudinal axis of the implant.

It is preferred that shelfs are formed from the ridges of the second thread by crossing of the channel-like structure. The shelfs are segments of the thread which have a cutting and an anchoring function. It is preferred if at least one shelf comprises at least one cutting projection. The term "cutting projection" is referring to means which are formed for facilitating a cutting process. This term comprises cutting blades but has a more general meaning.

It is further preferred if at least a part of the proximal, distal and / or intermediate sections comprises a cavity formed by an internal surface, wherein apertures are connecting the internal and external surface of the first element. The hollow structure of at least a part of the first element makes the implant lighter by weight than a comparable massive first element. It is further preferred that a mesh built by struts is formed in the cavity of the first element. The struts advantageously contribute to the stability of the otherwise hollow structure. The apertures and the mesh advantageously allow bony ingrowth through the apertures to the cavity. The cavity can be filled with one or more substances which encourage bony ingrowth. The second element can also comprise apertures. If the first and / or second element comprises apertures, the apertures are ideally not present in the shelf or in the threads, which have preferably a solid structure without apertures.

The inventive implant further preferably comprises means for accommodating a tool which is formed for driving the implant. The tool preferably comprises a tang, a sleeve, a locking knob, a sleeve knob, a latch, and a handle, wherein the tang has means for connecting to the proximal section of the first element, wherein the sleeve has means for connecting to the second element, wherein a first working position of the tool can be set by engaging the locking knob, and wherein a second working position can be set by releasing the locking knob.

In the first working position both the sleeve and the tang can be moved together by turning the handle. This advantageously facilitates a combined rotation of the first and second element of the inventive implant. In the second working position the first and second element can be moved by the tang and the sleeve, respectively, independently of each other, and only the second element is moved by turning the handle. The locking knob is released by use of the latch. The sleeve knob is provided for disconnecting the tool from the implant when necessary.

The means of the tang and the sleeve are ideally complementary to the means of the first and second element, respectively, so that they can be positively connected.

A second aspect of the present invention is related to a method for treating a joint comprising a first bone and a second bone, the method comprising the steps of:
providing an implant with a first element and a second element according to the present invention,
determining a trajectory of introducing the implant,
connecting the implant with a tool according to the present invention, wherein the first element of the implant is connected to a tang of the tool and the second element of the implant is connected to a sleeve of the tool,
setting the tool into a first working position with a move of the locking knob,
delivering the implant to a prepared site for introducing the implant so that the pointed head of the first element is contacting the first bone,
screwing the whole implant into the first bone and the second bone by applying force on the handle until the reservoir of the implant is contacting the second bone,
disengaging the locking knob using the latch, thereby setting the tool into a second working position,
screwing the second element into the first bone by applying force to the handle of the tool until the second element is completely within the first bone,
disconnecting the tool from the implant by engaging the sleeve knob.

The inventive method is advantageous for the same reasons as the inventive implant.

In a preferred embodiment of the inventive method the joint is a sacro-iliac joint, wherein the first bone is the ilium and the second bone is the sacrum.

Embodiments of the invention are described in conjunction with the accompanying figures.
- Figure 1: shows an implant according to the present invention.
- Figure 2: shows a head of a first element of the implant according to Fig. 1.
- Figure 3a: shows a longitudinal cut of the intermediate section of the implant according to Fig. 1
- Figure 3b: shows a cross section of the intermediate section of the implant according to Fig. 1
- Figure 4: shows the implant according to Fig. 1 as placed into two bones
- Figure 5: shows a tool for placing the implant according to Fig. 1.
- Figure 6: shows the tip of the tool according to Fig. 5.
- Figure 7: shows a flow chart of a method according to the present invention.

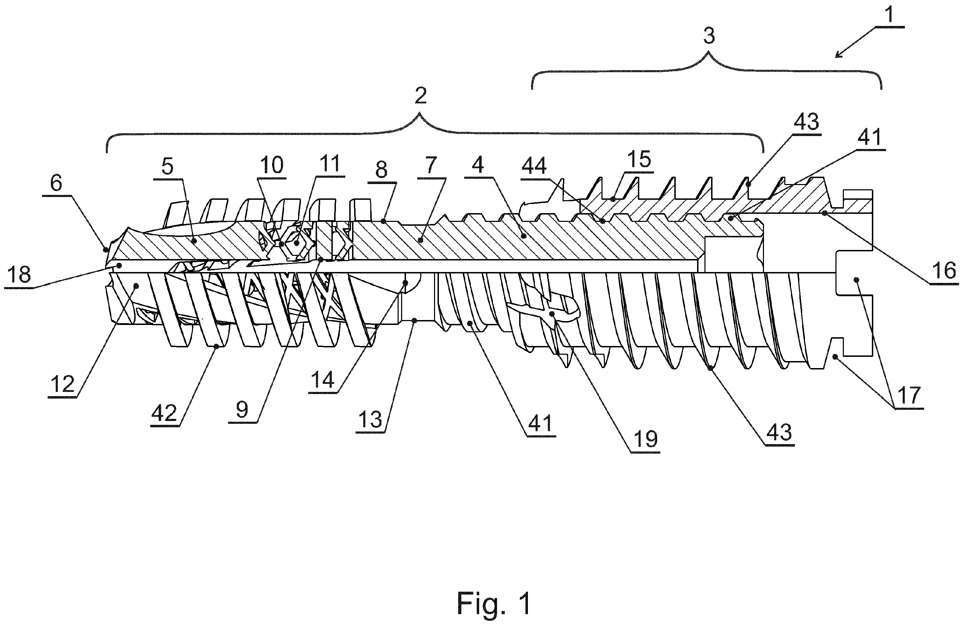

The sacroiliac implant 1 of this invention is shown in Fig. 1 as an elongate implant consisting of a first element 2 and a second element 3. The first element 1 has proximal section 4 and a distal section 5. The front end 6 of the distal section 5 is formed as a cutting head. An intermediate section 7 is located between the proximal 4 and the distal section 5. The first element 2 comprises an external surface 8 and an internal surface 9. The internal surface 9 is forming a contiguous surface defining a hollow structure, or cavity, within the proximal 4, distal 5, and intermediate section 7. The hollow structure of the first element 2 is stabilized by a mesh formed by a number of struts 10. A plurality of openings or apertures 11 is connecting the external 8 and internal surface 9. The openings or apertures 11 and the mesh of struts 10 provide conductive pathways by which bony ingrowth into the implant can be achieved.

In an embodiment of the inventive implant, the first element 2 may have a cavity with an internal surface in the distal section 5, but not in the intermediate section 7 and / or in the proximal section 4, so that apertures 11 would only be provided for the distal section 5. It is also possible that the intermediate section 7 and / or the proximal section 4 have a cavity and the distal section 5 is massive. Alternatively, the first element 2 may have a massive structure at all, so that no internal surface would be provided, and consequently no apertures.

The proximal part 4 of the first element 2 has a first thread 41, and the distal part 5 of the first element 2 has a second thread 42. Apertures are ideally not present in the threads.

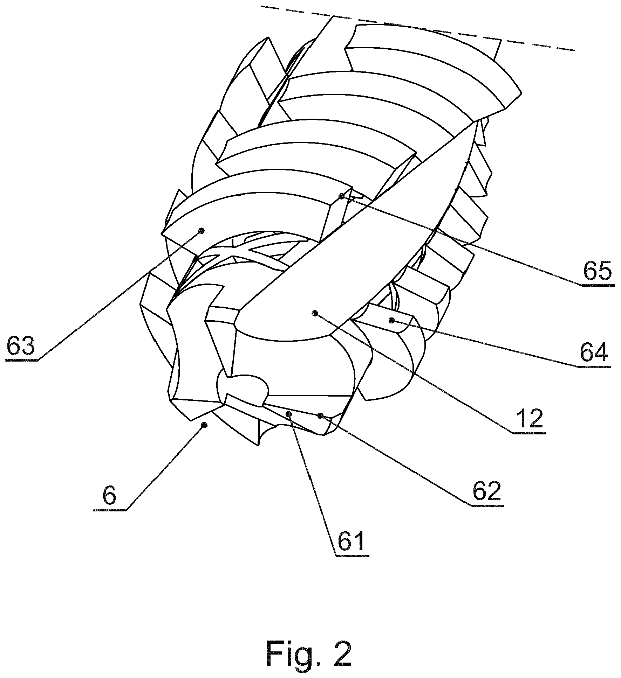

The front end 6 is shown in more detail in Fig. 2. The cutting head of the front end 6 is supporting its function of drilling and screwing the way of the implant into a bone upon an implantation process. A number of three cutting blades 61 are arranged at the front end 6. Alternatively, any suitable number of cutting blades 61 can be arranged which support the drilling of the first element 2 and thus of the whole implant 1 into the bone. Each cutting blade 61 has a cutting edge 62. A channel-like structure 12 starts immediately at the front end 6 which serves for transport of bone material away from the drilling site after it has been separated from the bone by the cutting blade 61. The starting point of the channel-like structure 12 is arranged relatively to the cutting blade 61 in such a way that material separated from the bone is received immediately by the channel-like structure 12. The channel-like structure 12 is formed with a roundish and even surface. The depth and width of the channel-like structure 12 are suited for a flow of bone material. The depth and width of the channel-like structure 12 are preferably the same over its length. Alternatively the dimensions of the channel-like structure 12 can also vary over its length, e.g. the channel-like structure 12 can be wider at the front end 6 and narrower in the proximity of the intermediate section 7. The path of the channel-like structure 12 is formed helically, but could alternatively also run parallel to the longitudinal axis of the first element 2. There are preferably three channel-like structures 12 arranged at the first element 2, however it is possible to arrange any suitable number of channel-like structures 12.

By crossing through the thread 42 the channel-like structure 12 is forming shelfs 63 from the thread, which is displayed in Fig. 2. At the crossing points of the channel-like structure 12, the brinks of the shelfs 63 are formed as cutting projections 64 at one side and as locking projections 65 at the other side.

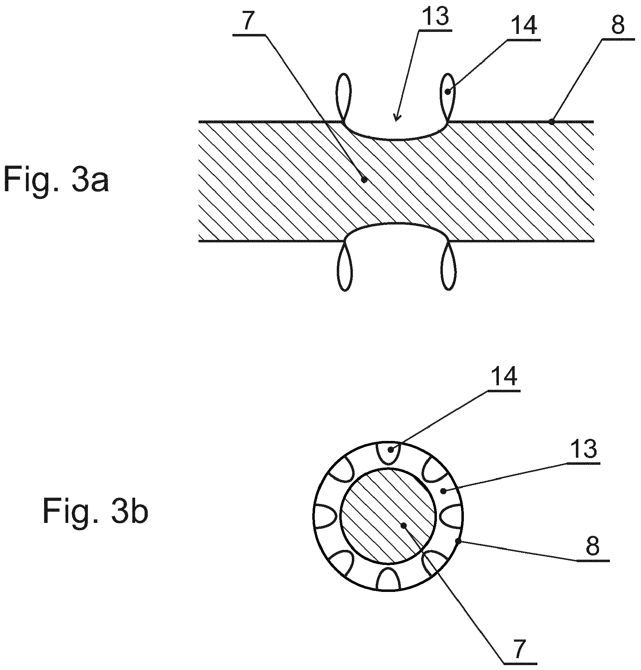

In Fig. 1 a reservoir 13 is shown which is formed in the external surface of the intermediate section 7. The reservoir 13 is a recess provided for receiving bone material which has been separated from the bone. The bone material is transported away from the front head 6 through the channel-like structure 12 which is running from the front end 6 to the reservoir 13. The reservoir 13 has the form of a hollow; it can be formed as a continuous hollow around the intermediate section or, alternatively, be divided into several smaller "sub"-reservoirs which appear as separate hollows in the external surface of the intermediate section 7. Means 14 are provided at the brinks of the reservoir 13 which support the dispersal of bone material from the reservoir 13 into the space between two bones the implant 1 is placed in. The means 14 are formed as projections which give the flow of bone material from the reservoir 13 a certain direction. The means 14 as shown in Fig. 3a and Fig. 3b have a roundish form with similarity to semicircles. Alternatively the means 14 could have a spiky form, or any other form suitable for supporting the dispersal of bone material. If the reservoir 13 is a continuous hollow, the means 14 are provided at both sides of the reservoir 13 and along its brinks around the intermediate section 7. If several small reservoirs are provided, means 14 can be provided around each reservoir.

The proximal part 4 of the first element 2 has a first thread 41. Apertures are ideally not present in the threads. The second element 3 comprises means 17 for accommodating a tool 30. A tunnel 18 for application of a guide wire is running through both the first 2 and the second element 3 of the implant.

The second element 3 as shown in Fig. 1 has an external surface 15 and an internal surface 16. The second element 3 can also have apertures (not shown). The second element 3 also comprises means 17 for accommodating a tool 30. A recess 19 is formed at the end of the second element 3 facing the intermediate section 7 of the first element 2. The recess 19 comprises cutting projections, such as cutting blades. Further cutting projections can be arranged at the same end of the second element 3. Bone material separated by the second element 3 will be received by the reservoir 13.

The external surface 15 has a third thread 43, and the internal surface 16 has a fourth thread 44. The pitch of the fourth thread 44 is of the same size as the pitch of the first thread 41, and the diameter of the internal surface 16 of the second element 3 is the same as the diameter of the external surface 8 of the proximal section 4 of the first element 2. The fourth thread 44 functions as an internal thread mating with the complementary first thread 41, so that this way the second element can be moved relatively to the first element by way of a screwing motion of the second element 3 around the first element 2. If the second element 3 has openings or apertures, they are not present in the threads.

The pitches of the second 42 and third thread 43 are identical. Moreover, the threads 42 and 43 are like-handed, meaning that they have the same direction of thread-advance.

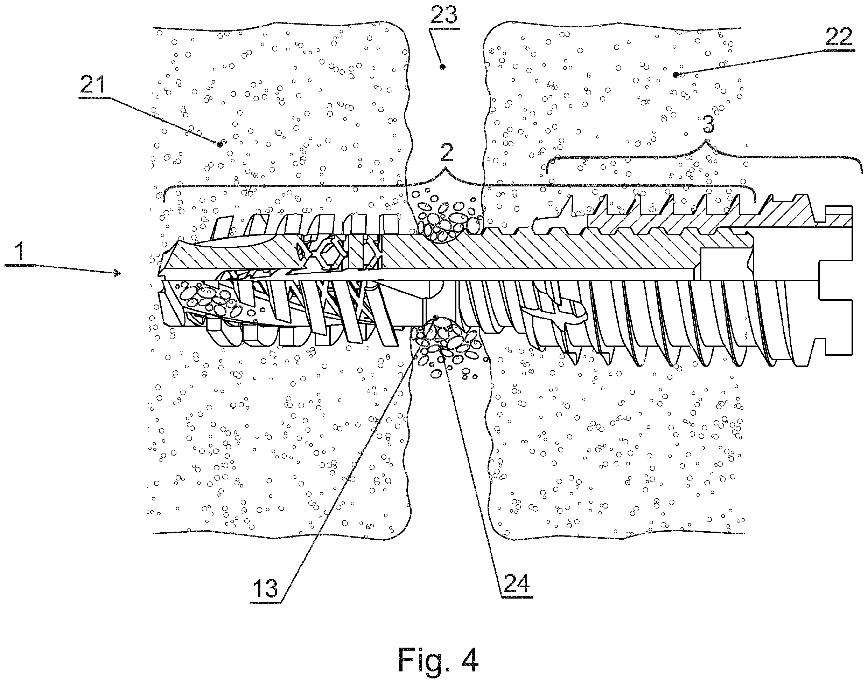

The position of the implant 1 within two adjacent bones is shown in Fig. 4. The first element 2 is placed into a first bone 21 and the second element 3 is placed into a second bone 22. If the implant 1 is a sacroilial implant, the first element 2 is placed into the sacrum and the second element 3 into the ilium. Bone material 24, removed from the first bone 21 upon the implantation process and stored in the reservoir 13, can be dispersed into the space 23 between the first bone 21 and the second bone 22.

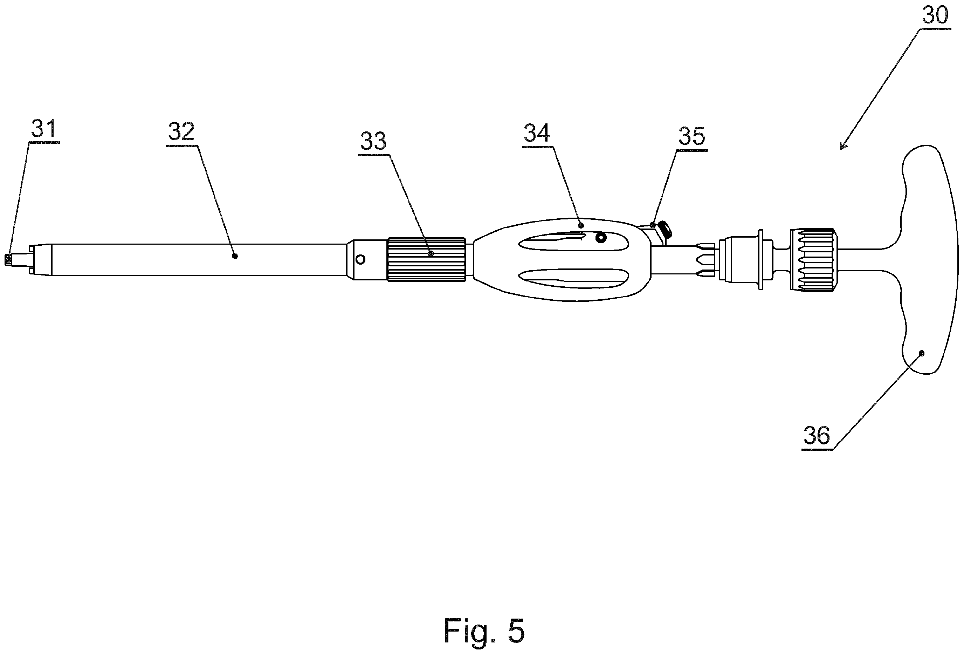

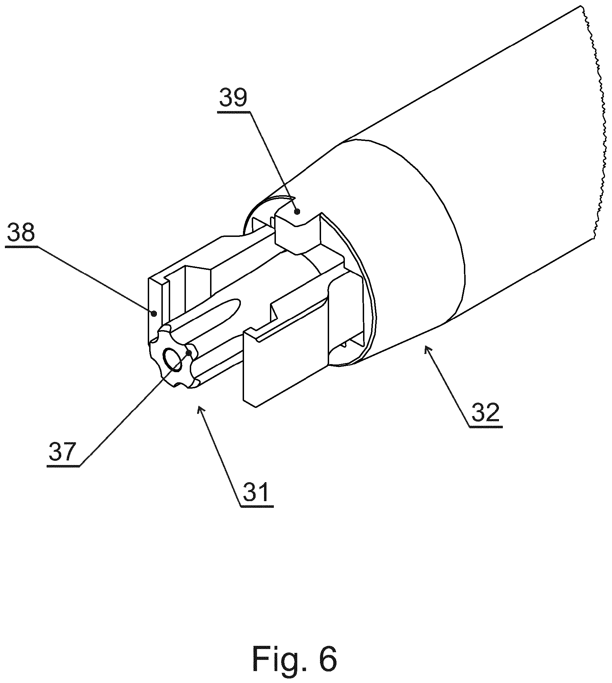

Fig. 5 shows a tool 30 for placing the implant into two adjacent bones. The tool 30 has a tang 31, a sleeve 32, a locking knob 33, a sleeve knob 34, a latch 35, and a handle 36. The long form of the tool 30 facilitates an efficient connection of the tang 31 to the first element 2 as well as of the sleeve 32 to the second element 3 of the implant 1. The tang 31 has connecting elements 37 which are provided for transmitting the torque from the tool 30 to the first element 2. As shown in Fig. 6, the connecting element 37 is formed as a five-pointed star. Alternatively it could also have other forms, for example an oval form, a square form, or a hexagonal form. Complementary forms are provided in the first element 2 for a positive connection of the tool 30 to the first element 2 of the implant 1. Additionally the tang 31 comprises forms like a clamp 38. The clamp 38 functions as an anchor in order to catch the first element 2.

The sleeve 32 has connecting elements 39 which are provided for connecting the tool 30 to the second element 3. As shown in Fig. 6, the connection element 39 has a square form. Alternatively, it could also have other forms, like a triangular or an oval form. Complementary forms are provided in the second element 3 for a positive connection of the tool 30 to the second element 3 of the implant 1.

The tang 31 is pushed by a spring mechanism which is operated by the handle 36. The tang 31 and the sleeve 32 are set into a first working position by engaging the locking knob 33. In the first working position of the tool 30, the tang 31 and the sleeve 32 are "locked", which means that they cannot be moved relatively to each other, and are both turned by rotational movement of the handle 36. The locking knob 33 can be disengaged by pushing the latch 35, setting the tool 30 in a second working position. In the second working position, the second element 3 can be moved relatively to the first element 2. The sleeve knob 34 is provided for disconnecting the tool 30 from the implant 1. Other working positions are possible, like a third working position in which only the tang 31 will be actively moved.

The implant 1 and the tool 30 form a system which is used for placing the implant 1 into two bones in order to stabilize a joint between the two bones.

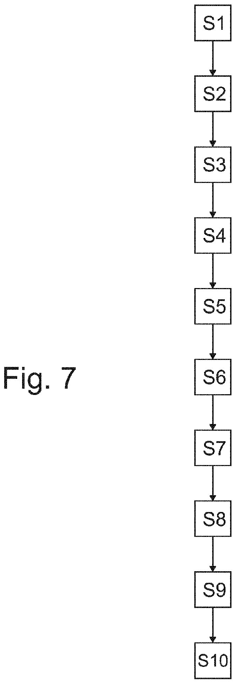

In a first step S1 of a method of placing an implant into a sacrum and an ilium, as shown in Fig. 7, an implant 1 is provided which has a first element 2 and a second element 3. The implant 1 is selected according to the size of the bones it shall be implanted in. In a second step S2 a trajectory of introducing the implant 1 into the bones is determined. It is possible to prepare a hole through the bones which is however smaller than the diameter of the first element 2. A Kirschner wire is inserted in the trajectory, which is also running through the tunnel 18 of the inventive implant 1. In a third step S3 a dilator is inserted. Steps S2 and S3 are carried out for preparation of the site and the bones for placing the implant 1. In a fourth step S4 the implant 1 is connected to a tool 30, wherein the first element 2 is connected to the tang 31 of the tool 30 and the second element 3 is connected to a sleeve 32 of the tool 30. In a fifth step S5 the tool 30 is set into a first working position by engaging the locking knob 33.

In a sixth step S6 the implant is delivered to the site prepared for introducing the implant so that the front end 6 of the first element 2 is contacting the ilium. In a seventh step S7 the first element 2 is introduced into the ilium and the sacrum by applying both a lateral force and a torque on the handle 36, so that the first element 2 is screwed into the bones, until the reservoir 13 of the implant 1 is contacting the sacrum.

In an eighth step S8 the locking knob 33 is disengaged using the latch 35, thereby setting the tool into a second working position. In a ninth step S9 the second element is screwed into the ilium by applying both a torque and a lateral force on the handle 36 of the tool until the second element is completely within the second bone. In a tenth step S10 the tool 30 is disconnected from the implant 1 by engaging the sleeve knob 34. The distance between ilium and sacrum can be set by how far the second element 3 is screwed onto the first element 2. Moreover, due to the fact that the pitch of the second thread 42 and the pitch of the third thread 43 are the same, the implant can be screwed out and screwed back in again at the same site, without disrupting the formed joint between ilium and sacrum.

### List of reference signs

- 1: = implant
- 2: = first element
- 3: = second element
- 4: = proximal part of first element
- 5: = distal part of first element
- 6: = front end
- 7: = intermediate section of first element
- 8: = external surface of first element
- 9: = internal surface of first element
- 10: = frame of first element
- 11: = aperture
- 12: = channel-like structure
- 13: = reservoir
- 14: = means for directing flow of material
- 15: = external surface of second element
- 16: = internal surface of second element
- 17: = means for accommodating a tool
- 18: = tunnel for guide wire
- 21: = first bone
- 22: = second bone
- 23: = space between first and second bone
- 24: = bone material
- 30: = tool
- 31: = tang
- 32: = sleeve
- 33: = locking knob
- 34: = sleeve knob
- 35: = latch
- 36: = handle
- 37: = connecting element of tang
- 38: = clamp
- 39: = connecting element of sleeve
- 41: = first thread
- 42: = second thread
- 43: = third thread
- 44: = fourth thread
- 61: = cutting blade
- 62: = cutting edge
- 63: = shelf
- 64: = cutting projection
- 65: = locking projection

## Claims

1. Implant (1) for immobilizing a joint between a first bone and a second bone, comprising at least a first element (2) and a second element (3), wherein the first element (2) has a cylindrical body comprising:
a proximal section (4) having a first thread (41),
a distal section (5) having a second thread (42), and
an intermediate section (7) interconnecting said proximal (4) and distal sections (5), wherein the proximal (4), distal (5) and intermediate section (7) have an external surface (8),
wherein the front end (6) of the distal section (5) is formed as a cutting head comprising at least one cutting blade (61) with at least one cutting edge (62),
wherein the second element (3) has a cylindrical body with an external surface (15) having a third thread (43) and an internal surface (16) having a fourth thread (44),
wherein the pitch of the fourth thread (44) is of the same size as the pitch of the first thread (41), and the diameter of the internal surface (16) of the second element (3) is the same as the diameter of the external surface (8) of the first element (2),
**characterized in that**
the second thread (42) and the third thread (43) have the same pitch.

2. Implant (1) according to claim 1, **characterized in that** the intermediate section (7) of the first element (2) has at least one recess in the external surface (8) forming a reservoir (13) suitable for bone material which is separated from a bone upon an implantation process.

3. Implant (1) according to claim 2, **characterized in that** the reservoir (13) in the intermediate section (7) comprises means (14) which are formed for directing a flow of material from the reservoir into a space between the first and the second bone.

4. Implant (1) according to claim 2 or 3, **characterized in that** the first element comprises at least one channel-like structure (12) which is running from the front end (6) to the reservoir (13).

5. Implant according to claim 4, **characterized in that** shelfs are formed from the ridges of the second thread (42) by crossing of the channel-like structure (12).

6. Implant according to claim 5, **characterized in that** at least one shelf comprises at least one cutting projection.

7. Implant (1) according to any of the foregoing claims, **characterized in that** at least a part of the proximal (4), distal (5) and / or intermediate sections (7) comprises a cavity formed by an internal surface (9), wherein apertures (11) are connecting the internal and external surface of the first element.

8. Implant (1) according to claim 6, **characterized in that** a mesh of struts is formed in the cavity of the first element.

9. Implant (1) according to any of the foregoing claims, **characterized in that** the first element (2) and the second element (3) both have means for accommodating a tool (30) which is formed for driving the implant (1).

10. Tool (30) having means for connecting to an Implant (1) according to any of the foregoing claims, **characterized in that** the tool comprises
a tang (31),
a sleeve (32),
a locking knob (33),
a sleeve knob (34),
a latch (35), and
a handle (36),
wherein the tang has means for connecting to the proximal section of the first element, wherein the sleeve has means for connecting to the second element, wherein a first working position of the tool (30) can be set by engaging the locking knob (33), and wherein a second working position can be set by releasing the locking knob (33).

11. Tool according to claim 10, **characterized in that** the means of the tang and the sleeve are complementary to the means of the first and second element, respectively.

12. Method for treating a joint with a first bone and a second bone, the method comprising the steps of
providing an implant with a first element and a second element according to claims 1 - 9,
determining a trajectory of introducing the implant,
connecting the implant with a tool which is formed for driving the implant, wherein the first element of the implant is connected to a tang of the tool and the second element of the implant is connected to a sleeve of the tool,
setting the tool into a first working position with a move of the locking knob,
delivering the implant to a prepared site for introducing the implant so that the pointed head of the first element is contacting the first bone,
screwing the whole implant into the first bone and the second bone by applying force on the handle until the reservoir of the implant is contacting the second bone,
disengaging the locking knob using the latch, thereby setting the tool into a second working position,
screwing the second element into the first bone by applying force to the handle of the tool until the second element is completely within the first bone,
disconnecting the tool from the implant by engaging the sleeve knob.

13. Method according to claim 12, wherein the joint is a sacro-iliac joint, wherein the first bone is the ilium and the second bone is the sacrum.
